# EUROPÄISCHE PATENTSCHRIFT

(11) **EP 3 520 850 B1**
(45) Veröffentlichungstag und Bekanntmachung des Hinweises auf die Patenterteilung: **10.03.2021**
(21) Anmeldenummer: 19401004.7
(22) Anmeldetag: 31.01.2019
(51) Int. Cl.: A61M 16/04, A61B 5/00, A61B 5/03, A61B 5/11, A61B 5/113, A61J 15/00, A61M 16/00, A61B 5/08, A61B 5/085, A61B 5/087, A61M 25/10

(54) **SYSTEM ZUR ERFASSUNG VON ATEMANSTRENGUNGEN EINES PATIENTEN**
SYSTEM FOR DETECTING A PATIENT'S RESPIRATORY EFFORT
SYSTÈME DE DÉTECTION DES EFFORTS RESPIRATOIRES D'UN PATIENT

(30) Priorität: 31.01.2018 DE 102018000757
(43) Veröffentlichungstag der Anmeldung: 07.08.2019
(73) Patentinhaber: Löwenstein Medical Technology S.A., 2557 Luxembourg (LU)
(72) Erfinder: Kremeier, Peter, 76149 Karlsruhe (DE)
(74) Vertreter: Marx, Thomas

(56) Entgegenhaltungen:
- EP-A1- 2 397 074
- WO-A1-2006/079152
- WO-A1-2010/121313
- DE-A1- 10 213 905
- DE-A1-102012 215 662
- US-A- 5 520 192
- US-A1- 2015 040 905

## Beschreibung

Die assistierte Beatmung ist eine unterstützende Beatmung. Ausgelöst wird die Beatmung durch einen sogenannten Trigger. Der Patient erzeugt am Beginn der Einatmung selbst einen Atemgas-fluss oder -druck, den das Beatmungsgerät als Trigger erkennt. Überschreitet der vom Patienten erzeugte Trigger die voreingestellte Schwelle, also das eingestellte Triggerniveau, so liefert das Beatmungsgerät Atemgas mit einem bestimmten Druck und/oder Volumen und erleichtert so den Atemzug.

Ziel einer assistierten Beatmung ist dabei, die Insufflation durch das Beatmungsgerat an die Atemanstrengungen des Patienten anzupassen, um dadurch den Patientenkomfort zu optimieren und die Atemarbeit zu minimieren. Eine Asynchronität zwischen dem Patienten und dem Beatmungsgerat, die als eine Diskrepanz zwischen der natürlichen Inspirationszeit des Patienten und der Insufflationszeit des Beatmungsgeräts definiert wird, ist ein häufiges Phänomen der klinischen Praxis. Fast ein Viertel der intubierten Patienten zeigen während einer assistierten mechanischen Beatmung erhebliche Asynchronitäten, die vielfach klinisch nicht erkannt werden. Das häufigste Asynchronitätsmuster sind ineffektive Triggerungen, bei denen die Inspirationsanstrengungen des Patienten keinen Beatmungshub triggern, da zum Zeitpunkt, zu dem versucht wird, den Trigger auszulosen, eine dynamische Hyperinflation besteht.

Eine Asynchronität kann einerseits ein Anzeichen für den Schweregrad des respiratorischen Status sein, kann jedoch andererseits auch in Verbindung mit ungeeigneten Einstellungen des Beatmungsgeräts, die die Dauer der mechanischen Beatmung verlängern, stehen. Verschiedene Einstellungen, die die Synchronität durch eine Verringerung der dynamischen Hyperinflation verbessern sollen, wurden bereits vorgeschlagen, wie beispielsweise die Anwendung eines externen positiven endexspiratorischen Drucks (PEEP) und die Verminderung der Insufflationszeit oder die Vermeidung einer inadäquat hohen Druck Unterstützung. Diese Ansätze sind jedoch bisher noch nicht systematisch verglichen worden, und ihre jeweiligen Auswirkungen auf die Atemarbeit und das Tidalvolumen bleiben somit unklar Nachfolgende Dokumente sind aus dem Stand der Technik bekannt DE 10 2012 215662; DE 102 13 905, EP 2 397 074, US 2015/040905, US 5 520 192, WO 2010/121313, WO 2006/079152.

Die Erfindung ist in den angehängten Ansprüchen definiert und betrifft ein System zur Erfassung von Atemanstrengungen eines Patienten, umfassend eine Druckermittlungsvorrichtung zum Ermitteln eines Drucks zu dem Zeitpunkt der Atemanstrengung des Patienten.

Die Atemanstrengung kann dabei eine aktive (muskuläre) oder eine passive (beispielsweise Rückstellkräfte) Atemanstrengung sein. Das System ist auch dadurch gekennzeichnet, dass der Zeitpunkt der Atemanstrengung der Beginn der Exspiration oder der Beginn der Inspiration des Patienten ist.

System zur Erfassung von Atemanstrengungen eines Patienten, umfassend eine Druckermittlungsvorrichtung zum Ermitteln eines Druckes zu dem Zeitpunkt der Atemanstrengung des Patienten.

Das System ist auch dadurch gekennzeichnet, dass die Druckermittlungsvorrichtung als Ösophagus-Katheter ausgebildet ist und einen gasgefüllten Ballon aufweist.

Das System ist auch dadurch gekennzeichnet, dass der Sensor des Beatmungsgerätes den Ösophagusdruck bestimmt, der über den gasgefüllten Ballon des Ösophagus-Katheters erfasst wird.

Das System ist alternativ oder ergänzend zur Erfassung von Atemanstrengungen eines Patienten ausgebildet, umfassend eine Druckermittlungsvorrichtung, zum Ermitteln eines Drucks, zu dem Zeitpunkt der Atemanstrengung des Patienten dadurch gekennzeichnet, dass die Druckermittlungsvorrichtung als Ösophagus-Katheter ausgebildet ist und einen gasgefüllten Ballon aufweist und umfassend ein Beatmungsgerät mit einem Druckeingangsstutzen für den Ösophagus-Katheter und einen Druck)Sensor, wobei eine Atemanstrengung des Patienten über den gasgefüllten Ballon des Ösophagus-Katheters erfasst wird.

Das System ist alternativ oder ergänzend dadurch gekennzeichnet, dass der transpulmonale Druck von dem Beatmungsgerät bestimmt wird unter Berücksichtigung des, vom Beatmungsgerät vorgegebenen, Beatmungsdrucks und dem sensorisch ermittelten Ösophagusdruck.

Das System ist alternativ oder ergänzend dadurch gekennzeichnet, dass der gasgefüllte Ballon einen Sensor aufweist, der eine Atemanstrengung registriert und an ein Beatmungsgerät übermittelt

Das System ist alternativ oder ergänzend dadurch gekennzeichnet, dass die Bestimmung des transpulmonalen Drucks am Ende der Ausatmung TPP ex und/oder am Ende der Einatmung TPP in durch einen bestimmten Messvorgang erfolgt, bei dem das Beatmungsgerät einen Atemgastransport zu oder von dem Patienten verhindert.

Das System ist alternativ oder ergänzend dadurch gekennzeichnet, dass eine Atemanstrengung des Patienten mit zumindest einem hinterlegten Schwellwert verglichen wird und bei Überschreitung des Schwellwertes einem Trigger entspricht.

Das System ist alternativ oder ergänzend dadurch gekennzeichnet, dass der Zeitpunkt der Beginn der Exspiration oder der Beginn der Inspiration des Patienten ist.

Das System ist alternativ oder ergänzend dadurch gekennzeichnet, dass die Druckermittlungsvorrichtung den Ösophagusdruck fortlaufend, beispielsweise auch zu Beginn der Inspiration oder der Exspiration bestimmt.

Das System ist alternativ oder ergänzend dadurch gekennzeichnet, dass das System zudem eine Einrichtung, beispielsweise eine Steuereinheit, umfasst die den, von dem Beatmungsgerät bereitgestellten, Atemgasdruck unter Berücksichtigung des ermittelten Ösophagusdrucks oder transpulmonalen Drucks vorgibt.

Das System ist alternativ oder ergänzend dadurch gekennzeichnet, dass die Einrichtung bei Überschreiten oder Unterschreiten eines Schwellwertes für den Ösophagusdruck ein Steuersignal für das Beatmungsgerät erzeugt, zur Vorgabe eines inspiratorischen oder exspiratorischen Atemgasdrucks.

Das System ist alternativ oder ergänzend dadurch gekennzeichnet, dass die Steuereinheit des Beatmungsgerätes Atemgasparameter (Druck, Fluss, Volumen, Frequenz) für eine kontrollierte oder assistierte Beatmung bereitgestellt und die Atemgasquelle zur Vorgabe der kontrollierten oder assistierten Beatmung ansteuert.

System nach zumindest einem der vorhergehenden Ansprüche dadurch gekennzeichnet, dass aus der Ösophagusdruckkurve die Anzahl der Inspirationsbemühungen des Patienten pro Zeiteinheit) erkannt und aufgezeichnet wird.

Das System ist alternativ oder ergänzend dadurch gekennzeichnet, dass aus der Beatmungsdruckkurve oder der Steuereinheit die Anzahl der vorgegebenen Inspirationen pro Zeiteinheit) erkannt und aufgezeichnet wird.

Das System ist alternativ oder ergänzend dadurch gekennzeichnet, dass die Inspirationsbemühungen des Patienten pro Zeiteinheit mit den vorgegebenen Inspirationen pro Zeiteinheit verglichen und so der Grad der Synchronisierung zwischen den Inspirationsbemühungen des Patienten und der Inspiratorischen Vorgabe des Beatmungsgeräts ermittelt wird

Das System ist alternativ oder ergänzend dadurch gekennzeichnet, dass diesem Vergleich ein Index der Fehltriggerungen entnommen wird, der den Grad der Synchronisierung zwischen den Inspirationsbemühungen des Patienten und der Inspiratorischen Vorgabe des Beatmungsgeräts repräsentiert.

Das System ist alternativ oder ergänzend dadurch gekennzeichnet, dass die Einrichtung zur Erfassung der Synchronität Fehltrigger, aus dem zeitlichen Abstand zwischen dem vorgegebenen Atemgashub und der Atemanstrengung des Patienten, erkennt und eine Fehltriggerrate oder ein Index ermittelt und speichert oder darstellt.

Das System ist alternativ oder ergänzend dadurch gekennzeichnet, dass die Einrichtung zur Erfassung der Synchronität Fehltrigger, aus dem zeitlichen Abstand zwischen dem vorgegebenen Atemgashub und der Atemanstrengung des Patienten, dann erkennt, wenn der zeitliche Abstand größer ist als /Sekunde, bevorzugt größer ist /Sekunde, besonders bevorzugt größer ist als eine Sekunde.

Das System zur Erfassung von Atemanstrengungen eines Patienten, umfasset alternativ oder ergänzend eine Druckermittlungsvorrichtung, zum Ermitteln eines Drucks, zu dem Zeitpunkt der Atemanstrengung des Patienten dadurch gekennzeichnet, dass die Druckermittlungsvorrichtung als Ösophagus-Katheter ausgebildet ist und einen gasgefüllten Ballon aufweist und umfassend ein Beatmungsgerät mit einem Druckeingangsstutzen für den Ösophagus-Katheter und einen Druck)Sensor, wobei eine Atemanstrengung des Patienten über den gasgefüllten Ballon des Ösophagus-Katheters erfasst wird, wobei das System zudem eine Einrichtung zur Erfassung der Synchronität eines durch das Beatmungsgerät (zeitlich) vorgegebenen Atemgashubs (Druck oder Volumen oder Fluss) mit der Atemanstrengung des Patienten, umfasst, wobei diesem Vergleich ein Index der Fehltriggerungen entnommen wird, der den Grad der Synchronisierung zwischen den Inspirationsbemühungen des Patienten und der Inspiratorischen Vorgabe des Beatmungsgeräts repräsentiert.

Das System ist alternativ oder ergänzend dadurch gekennzeichnet, dass die Einrichtung bei erkannten Fehltriggern ein Steuersignal für das Beatmungsgerät erzeugt, zur Vorgabe einer veränderten Einatemzeit und/oder eines veränderten PEEP und/oder einer veränderten Triggerempfindlichkeit.

Das System ist alternativ oder ergänzend dadurch gekennzeichnet, dass der Ösophagus-Katheter den funktionellen positiven endexspiratorischen Druck, welcher als intrinsischen PEEP PEEPi) bezeichnet wird, ermittelt.

Das System ist alternativ oder ergänzend dadurch gekennzeichnet, dass die Einrichtung bei Überschreiten oder Unterschreiten eines Schwellwertes für den PEEPi ein Steuersignal für das Beatmungsgerät erzeugt, zur Vorgabe eines veränderten Atemgasvolumens oder einer veränderten Ausatemzeit.

Das System ist alternativ oder ergänzend dadurch gekennzeichnet, dass eine Einrichtung zur Erfassung des optimalen Füllvolumens des Ballons umfasst ist, wobei die Einrichtung das geringste Füllvolumen bestimmt, welches die größte Pulsdruckvariation "Swing") des Ballons in einem Atemzug von inspiratorisch zu exspiratorisch) ausmacht. Die Einrichtung zur Erfassung des optimalen Füllvolumens des Ballons kann ein Fluss oder Volumensensor sein, der beispielsweise in dem Beatmungsgerät angeordnet ist und mit der Steuereinheit verbunden ist.

Die Erfindung umfasst auch ein System mit einer Einrichtung zur Erfassung des optimalen Füllvolumens des Ballons, wobei die Einrichtung das Füllvolumen bestimmt, welches die größte Pulsdruckvariation ("Swing") des Ballons in einem Atemzug (von inspiratorisch zu exspiratorisch) ausmacht.

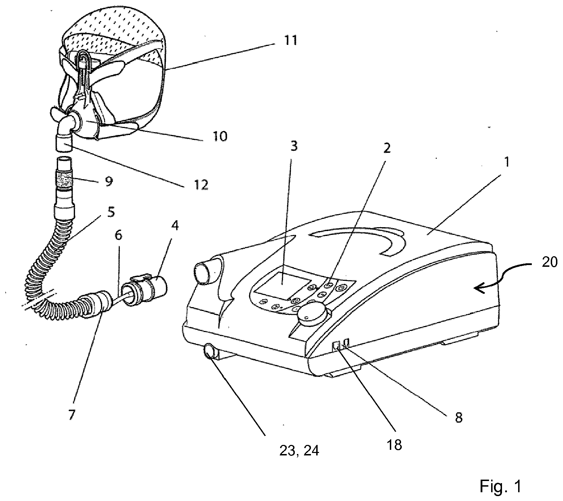

Fig. 1 zeigt den grundsätzlichen Aufbau eines Beatmungsgerätes (20). Im Bereich eines Gerätegehäuses (1) sind ein Bedienelement (2) und/oder ein Bedien-und Informationssystem (3) angeordnet. Über eine Kopplung (4) wird ein Verbindungsschlauch (5) angeschlossen. Entlang des Verbindungsschlauches (5) kann ein zusätzlicher Druckmessschlauch (6) verlaufen, der über einen Druckeingangsstutzen (7) mit dem Gerätegehäuse (1) verbindbar ist. Zur Ermöglichung einer Datenübertragung weist das Gerätegehäuse (1) zumindest eine Schnittstelle (8,18) auf. Ein Anfeuchter (21) oder ein Vernebler (22) kann zudem adaptiert werden. Das Beatmungsgerät weist eine Atemgasquelle (17) auf
Im Bereich einer dem Gerätegehäuse (1) abgewandten Ausdehnung des Verbindungsschlauches (5) ist beispielhaft ein Ausatmungselement (9) angeordnet. Ebenfalls kann ein Ausatemventil verwendet werden.

Das Beatmungsgerät (20) kann als Schlaftherapiegerät, als High-Flow-Gerät, als Anästhesiegerät, als klinisches- oder Heim- oder Notfall-Beatmungsgerät ausgebildet sein.

Fig. 1 zeigt darüber hinaus ein als Beatmungsmaske (10) ausgebildetes Patienten-Interface. Eine Fixierung im Bereich eines Kopfes eines Patienten kann über eine Kopfhaube (11) erfolgen. Im Bereich seiner dem Verbindungsschlauch (5) zugewandten Ausdehnung weist das Patienten-Interface (10) ein Kupplungselement (12) auf. Das Patienten-Interface kann beispielsweise auch als Tubus ausgebildet sein.

Über die Schnittstelle (8,18) kann die Eingabe und / oder Ausgabe von Daten, wie beispielsweise Totraumvolumen, erfolgen. Die Schnittstellen können kabelgebunden, als Infrarot-Schnittstelle, als Bluetooth-Schnittstelle oder USB realisiert sein. Bevorzugt ist auch ein Kartenschacht vorgesehen. Die Schnittstelle (8) kann auch als LAN-Schnittstelle oder als sonstige Schnittstelle zur Anbindung an das Internet ausgeführt sein. Im Bereich eines Gerätegehäuses kann ein Sauerstoffzuschaltventil an die Vorrichtung zur Beatmung adaptiert werden. Es ist denkbar, das Atemgas zusätzlich mit Sauerstoff anzureichern, um die Patientenversorgung zu verbessern.

Das erfindungsgemäße Beatmungsgerät (20) ist so ausgelegt, dass es über einen Schlauch und ein Patienteninterface mit einem Patienten verbunden werden kann, um eine Beatmung bereitzustellen. Es umfasst eine Quelle für Atemgas (17), die beispielsweise als ein Elektromotor mit Gebläserad oder als Druckgasanschluss mit zumindest einem Ventil ausgebildet ist. Das Beatmungsgerät weist eine Einrichtung zur Ermittlung von Druck und/oder Fluss und/oder Volumen des Atemgases auf. Eine Steuereinheit (19) ist so ausgelegt, dass sie beispielsweise für jeden Atemzyklus auf der Basis eines vorbestimmten Wertes und/oder auf Basis von Messsignalen für die Parameter Druck und/oder Flow und/oder Volumen einen Atemgasparameter bestimmt und die Quelle für Atemgas derart reguliert, dass der Atemgasparameter appliziert wird. Die Steuereinheit kann die Parameter der Beatmung kontrolliert vorgeben und/oder zumindest teilweise assistiert oder adaptiv unter Berücksichtigung von Messignalen.

Die Steuereinheit (19) ist beispielsweise so ausgelegt, dass sie den aktuellen Druck und/oder Flow und/oder das Volumen von Atemgas bestimmt und den aktuellen Wert über das mit der Steuereinheit Bedien- und Informationssystem oder der Anzeige (3) darstellt. Die Steuereinheit (19) ist außerdem so ausgelegt, dass sie bezogen auf einen oder mehrere Parameter Trendveränderungen ihrer Berechnungen über der Zeit bestimmt, wobei die Trendveränderungen auf der Anzeige angezeigt werden können.

Weiterhin vergleicht die Steuereinheit (19) solche ParameterWerte, die von einem Benutzer vorgegeben wurden, beispielsweise obere und untere Druckgrenzen oder eine maximal tolerierbare Anzahl von Apnoen pro Zeiteinheit, oder eine maximal tolerierbare Leckage, mit den aktuellen Werten und generiert eine BenutzerInformation zu Abweichungen von der Vorgabe. Die Benutzerinformation wird bevorzugt über das Bedien- und Informationssystem (3) graphisch visualisiert.

Die Steuereinheit (19) ist beispielsweise auch so ausgelegt, dass sie den Ösophagusdruck zumindest zeitweise oder phasenweise bestimmt. Das Beatmungsgerät (20) weist dazu einen (pneumatischen oder elektronischen oder optischen) Druckmesseingang und einen Drucksensor (23, 24) auf, an den ein Ösophagusballon (62) zumindest mittelbar angeschlossen ist.

Die Steuereinheit (19) ist beispielsweise eingerichtet und ausgebildet eine Veränderung des Ösophagusdruckes zu identifizieren und daraufhin das Beatmunggerät zur Vorgabe eines Beatmungsparameters anzusteuern.

Bei Überschreiten oder Unterschreiten eines Schwellwertes für den Ösophagusdruck (60) generiert die Steuereinheit (19) beispielsweise ein Steuersignal für das Beatmungsgerät (20), zur Vorgabe eines inspiratorischen oder exspiratorischen Atemgasdrucks. Bei Überschreiten oder Unterschreiten eines Schwellwertes für den Ösophagusdruck (60) generiert die Steuereinheit (19) beispielsweise alternativ ein Steuersignal für das Beatmungsgerät (20), zur Beendigung der Vorgabe eines inspiratorischen oder exspiratorischen Atemgasdrucks.

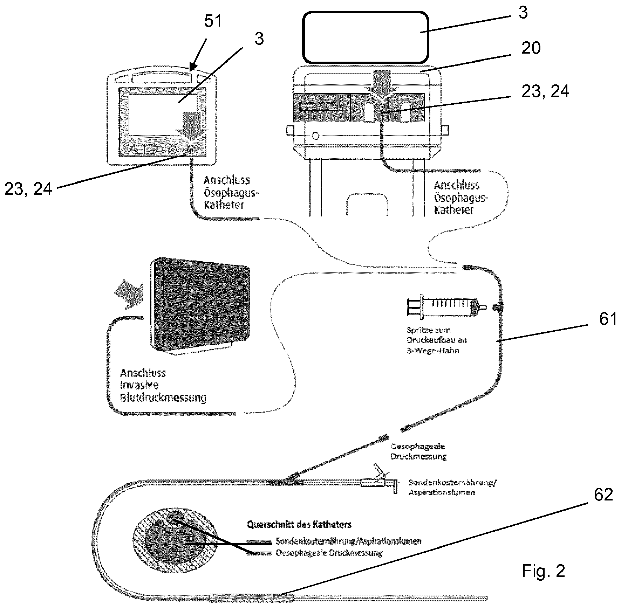

Fig. 2 zeigt schematisch die Anordnung Beatmungsgerätes (20) in dem System. Die bettseitige Messung des Ösophagusdrucks (60, Peso) basiert auf der Verwendung eines Ösophagusballons (62) und spiegelt dabei als Surrogatparameter die Änderungen des Pleuraldrucks (63) wider. Die fortlaufend oder phasenweise gemessenen bzw. bestimmten transpulmonalen Drücke (64) erlauben, die mechanische Druck- und Volumenbelastung unter Beatmung zu bewerten und die Beatmung entsprechend lungenprotektiv zu adaptieren. Der transpulmonale Druck (64) wird dazu aus dem gemessenen Ösophagusdrucks (60, Peso) und dem vom Beatmungsgerät (20) vorgegebenen oder gemessenen Beatmungsdrucks (27) rechnerisch bestimmt oder ermittelt. Erst die Messung des Ösophagusdrucks (60, Peso), ermöglicht die Bestimmung des transpulmonalen Drucks (64). Dieser ist derjenige Druck, der für die Dehnung der Lunge und der Brustwand erforderlich ist.
Der transpulmonale Druck (64) entspricht dem Druckunterschied zwischen den Alveolen und dem Ösophagus. Beispielsweise kann der transpulmonale Druck (64) bei endinspiratorischer oder endexspiratorischer Okklusion gemessen werden kann.
Das System zur Erfassung von Atemanstrengungen eines Patienten (40), umfasst daher eine Druckermittlungsvorrichtung, die als Ösophagus-Katheter (61) ausgebildet ist, zum Ermitteln eines transpulmonalen Druckes (64) beispielsweise unter Anwendung endinspiratorischer oder endexspiratorischer Okklusionsmanöver. Der Ösophagus-Katheter (61) weist einen luftgefüllten Ballon (62) auf, der als Druckaufnehmer dient. Das Beatmungsgerät (20) weist einen Druckeingangsstutzen (23) für den Ösophagus-Katheter (61) und einen entsprechenden Drucksensor (24) auf, der den Ösophagusdruck (60, Peso) bestimmt.
Alternativ oder ergänzend kann der Ösophagus-Katheter (61) an einen separaten Monitor (51) angeschlossen werden. Der Monitor weist dazu einen (pneumatischen oder elektronischen oder optischen) Druckmesseingang und einen Drucksensor (23, 24) auf, an den ein Ösophagusballon (62) zumindest mittelbar, über den Ösophagus-Katheter (61), angeschlossen ist. Der Monitor (51) und/oder das Beatmungsgerät (3) kann den Druckverlauf des transpulmonalen Druckes (64) und/oder Ösophagusdrucks (60, Peso) und/oder des Beatmungsdruckes (27) darstellen und aufzeichnen. Der Ösophagus-Katheter (61) weist im einfachsten Fall zumindest ein Lumen für die Druckmessung auf und einen Ösophagusballon (62). Der Ösophagus-Katheter (61) kann alternativ oder ergänzend mehrlumig ausgeführt sein und so zudem eine Sondenernährung ermöglichen. Optional kann zusätzlich eine Blutdruckmessung und/oder eine Temperaturmessung ergänzt werden.

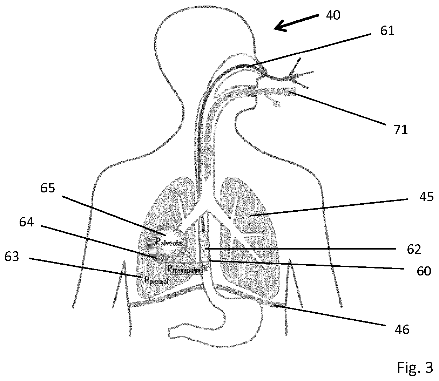

Fig. 3 zeigt schematisch die Anordnung des Systems im Patienten. Das System zur Erfassung von Atemanstrengungen eines Patienten (40), umfasst eine Druckermittlungsvorrichtung, die als Ösophagus-Katheter (61) ausgebildet ist, zum Ermitteln eines transpulmonalen Drucks (64). Dieser gleicht dem alveolären Druck in guter Näherung. Der transpulmonale Druck wird bestimmt aus dem (vom Beatmungsgerät vorgegebenen) Beatmungsdruck (27) und dem Peso (60). Die Messung und Bestimmung erfolgt beispielsweise am Ende der Ausatmung (64 TPP ex) und/oder am Ende der Einatmung (64 TPP in) durch einen bestimmten Messvorgang.
Der Ösophagus-Katheter (61) weist einen luftgefüllten Ballon (62) auf, der als Druckaufnehmer dient. Der Ballonabschnitt liegt dabei benachbart zu der Lunge. Ein Beatmungstubus (71) kann in der Luftröhre platziert werden, um den Patienten zu beatmen. Zu erkennen sind die beiden Lungenflügel (45) und das Zwerchfell (46).

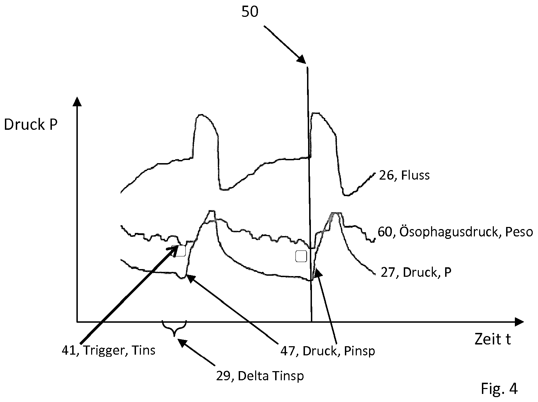

Fig. 4 zeigt schematisch den Verlauf der Signale Fluss (26), Ösophagus-Druck (60) und Beatmungsdruck (27). Aus dem Verlauf der Signale ist zu erkennen, dass die Druckermittlungsvorrichtung den Ösophagusdruck (60) bestimmt. Eine Atemanstrengung des Patienten (41) wird als Ausschlag in dem Ösophagusdruck-Signal erkannt. Zwerchfellkontraktionen erzeugten so beispielsweise einen negativen Peso-Druck (bei 41). Dieser Patienten-Trigger (41) wird, im linken Teil der Fig. 4, vom Beatmungsgerät nicht als Trigger für die inspiratorische Druckerhöhung (47) erkannt. Dieses wird deutlich durch die zeitliche Differenz (29) zwischen dem Patienten-Trigger (41) und der tatsächlichen inspiratorischen Druckerhöhung (47) durch das Beatmungsgerät. Erfindungsgemäß kann diese die zeitliche Differenz (29) zwischen dem Patienten-Trigger (41) und der tatsächlichen inspiratorischen Druckerhöhung (47) durch das Beatmungsgerät erkannt und ausgewertet werden, um eine Asynchronität (30) zwischen dem Patienten und dem Beatmungsgerät aufzuzeigen, beispielsweise als Index vergeblicher Patienten-Trigger (41).

Erfindungsgemäß ist auch daran gedacht, dass eine Atemanstrengung des Patienten (41) als Ausschlag in dem Ösophagusdruck-Signal erkannt wird. Zwerchfellkontraktionen erzeugten so beispielsweise einen negativen Peso-Druck (bei 41). Dieser Patienten-Trigger (41) wird, im rechten Teil der Fig. 4, zum Zeitpunkt (50) vom Beatmungsgerät als Trigger für die inspiratorische Druckerhöhung (47) erkannt. Die Einrichtung (19, Steuereinheit) zur Vorgabe des Atemgasdrucks hat hier ein Überschreiten oder Unterschreiten eines Schwellwertes für den Ösophagusdruck (60) erkannt und generiert ein Steuersignal für das Beatmungsgerät (20) zur Vorgabe eines inspiratorischen Atemgasdrucks (47). Der inspiratorische Atemgasdruck (47) führt zu einem Atemgasfluss (26) zu dem Patienten.

Mit der Ösophagusdruckmessung steht ein Messverfahren zur Verfügung, welches eine Patient-Respirator-Asynchronität demaskieren kann. Die Überwachung des Ösophagusdruckes (60) hilft beim Erkennen und Behandeln der Ursachen der unwirksamen Patientenanstrengungen (41). Zwerchfellkontraktionen erzeugten einen negativen Peso-Druck, auf denen keine inspiratorische Reaktion in der Beatmungsdruckkurve folgt. Durch die Kombination von hohem Atemantrieb, hoher Flussrate und niedrigem Tidalvolumen tritt diese Problemkonstellation häufiger auf. Eine bettseitige Überwachung der Atemmuskelaktivität in Echtzeit mittels Ösophagusdruck erlaubt den Grad der Synchronisierung (30) zwischen den Inspirationsbemühungen des Patienten und der Insufflationszeit des Beatmungsgeräts zu bewerten und die Beatmungsparameter (z.B. Optimierung der Insufflationszeit oder der Druck Unterstützung, bzw. des PEEPs) oder die Triggerempfindlichkeit entsprechend anzupassen.

Eine getriggerte Beatmung kann eine Zwerchfelldysfunktion vermeiden, indem es dem Patienten ermöglicht wird, spontane Inspirationsanstrengungen zu erzeugen. Ziel einer assistierten Beatmung ist dabei, die Insufflation durch das Beatmungsgerät (47) an die Atemanstrengungen des Patienten (41) anzupassen, um dadurch den Patientenkomfort zu optimieren und die Atemarbeit zu minimieren. Eine Asynchronität (30) zwischen dem Patienten und dem Beatmungsgerät, die als eine Diskrepanz zwischen der natürlichen Inspirationszeit des Patienten und der Insufflationszeit des Beatmungsgeräts definiert wird, ist ein häufiges Phänomen der klinischen Praxis. Fast ein Viertel der intubierten Patienten zeigen während einer assistierten mechanischen Beatmung erhebliche Asynchronitäten, die vielfach klinisch nicht erkannt werden. Das häufigste Asynchronitätsmuster sind ineffektive Triggerungen (41), bei denen die Inspirationsanstrengungen des Patienten keinen Beatmungshub triggern, da zum Zeitpunkt, zu dem versucht wird, den Trigger auszulösen, beispielsweise eine dynamische Hyperinflation besteht.
Eine Asynchronität (29) kann einerseits ein Anzeichen für den Schweregrad des respiratorischen Status sein, kann jedoch andererseits auch in Verbindung mit ungeeigneten Einstellungen des Beatmungsgeräts, die die Dauer der mechanischen Beatmung verlängern, stehen. Verschiedene Einstellungen, die die Synchronität durch eine Verringerung der dynamischen Hyperinflation verbessern sollen, wurden bereits vorgeschlagen, wie beispielsweise die Anwendung eines externen positiven endexspiratorischen Drucks (PEEP) und die Verminderung der Insufflationszeit oder die Vermeidung einer inadäquat hohen Druckunterstützung. Diese Ansätze sind jedoch bisher noch nicht systematisch verglichen worden, und ihre jeweiligen Auswirkungen auf die Atemarbeit und das Tidalvolumen bleiben somit unklar. Mit der Ösophagusdruckmessung steht ein Messverfahren zur Verfügung, welches eine Patient-Respirator-Asynchronität demaskieren kann. Die Überwachung des Ösophagusdruckes hilft beim Erkennen und Behandeln der Ursachen der unwirksamen Patientenanstrengungen. Weil diese Asynchronität mit einer verlängerten Dauer der mechanischen Beatmung verbunden ist, könnte sich dies potenziell auf die Dauer der maschinellen Beatmung auswirken. Zwerchfellkontraktionen erzeugten einen negativen Peso-Druck, auf denen keine inspiratorische Reaktion in der Beatmungsdruckkurve folgt. Durch die Kombination von hohem Atemantrieb, hoher Flussrate und niedrigem Tidalvolumen tritt diese Problemkonstellation häufiger auf. Eine bettseitige Überwachung der Atemmuskelaktivität in Echtzeit mittels Ösophagusdruck erlaubt den Grad der Synchronisierung zwischen den Inspirationsbemühungen des Patienten und der Insufflationszeit des Beatmungsgeräts zu bewerten und die Beatmungsparameter (z.B. Optimierung der Insufflationszeit oder der Druckunterstützung, bzw. des PEEPs) entsprechend, beispielsweise automatisiert, anzupassen.

Aus der Ösophagusdruckkurve (60) wird die Anzahl der Inspirationsbemühungen (41) des Patienten pro Zeiteinheit erkannt und aufgezeichnet.

Zudem wird aus der Beatmungsdruckkurve (27) oder der Steuereinheit die Anzahl der vorgegebenen Inspirationen (47) pro Zeiteinheit erkannt und aufgezeichnet.

Werden die Inspirationsbemühungen des Patienten (41) pro Zeiteinheit mit den vorgegebenen Inspirationen (47) pro Zeiteinheit verglichen, kann der Grad der Synchronisierung (30) zwischen den Inspirationsbemühungen (41) des Patienten und der Inspiratorischen Vorgabe des Beatmungsgeräts ermittelt werden.

Erfindungsgemäß kann diesem Vergleich ein Index (30) der Fehltriggerungen entnommen werden, der den Grad der Synchronisierung (30) zwischen den Inspirationsbemühungen des Patienten und der Inspiratorischen Vorgabe des Beatmungsgeräts repräsentiert.

**Tab.1: Demaskierung von Patienten-Respirator-Asynchronie**

| **Fallszenarium** | **Klinische Bedeutung** | **Klinische Empfehlung** |
|---|---|---|
| Keine Peso Drucknegativierung vor der Druckabgabe durch das Beatmungsgerät bei spontanatmenden Patienten | Autotriggerung | Kontrolle & ggfs. Adaption: |
| | | • Leckagen |
| | | • Triggereinstellungen |
| | | • Kondensat in den Schläuchen |
| | | • Schlauchsysteme & Beatmungszubehör mit hoher Resistance |
| | | • Sedierungstiefe |
| Peso-Drucknegativierung ohne Druckabgabe durch das Beatmungsgerät bei spontanatmenden Patienten | Asynchronität | Kontrolle & ggfs. Adaption: |
| | | • Leckagen |
| | | • Triggereinstellungen |
| | | • Druckanstiegsgeschwindigkeit ("Rampe") |
| | | • Inspiratorische Terminierung ("Flowabschaltkriterium") |
| | | • PEEP |
| | | • Kondensat in den Schläuchen |
| | | • Schlauchsysteme & Beatmungszubehör mit hoher Resistance |
| Peso-Inspirationszeit länger als Inspirationszeit des Beatmungsgeräts | Doppel-Triggerung oder vorzeitiges Ende der Inspirationsph ase | Kontrolle & ggfs. Adaption: |
| | | • Inspirationszeit Beatmungsgerät |
| | | • Höhe und Art der Druckunterstützung |
| | | • Metabolische Azidose |
| | | • Enzephalopathie |
| Periodisch auftretende Peso-Negativierung nach der Abgabe von mandatorischen Atemzüge | Erkennung von "Reverse Triggering" | • Sedierung reduzieren oder ändern (inadäquate Sedierungstiefe) |

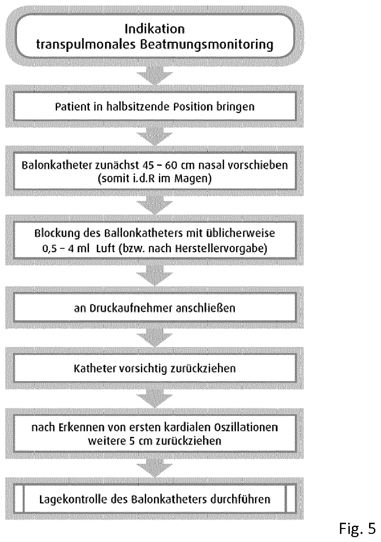

Fig. 5 zeigt die Platzierung des Peso-Katheters. Bei aufrecht sitzenden Probanden wurde der Pleuraldruck (63) durch Messen des Ösophagusdrucks unter Verwendung eines ösophagealen Ballonkatheters geschätzt. Die PESO-Änderungen während des Atemzyklus spiegeln die Änderungen des auf die Lungenoberflache einwirkenden Pleuraldrucks (63) wider. Der Unterschied zwischen Beatmungs- und Ösophagusdruck ist eine gültige Schätzung des transpulmonalen Drucks (64, TPP) im Bereich um den Ballonkatheter. Absolute Werte von Peso können von der Atemmechanik, dem Lungenvolumen, dem Gewicht des Mediastinums, dem Abdomen, der Haltung, der Reaktionsfähigkeit der glatten Muskelwand und den mechanischen Eigenschaften des Ballons beeinflusst werden.

Der aktuelle Kenntnisstand über die Auswirkung der Position des Patienten auf den beobachteten Ösophagusdruck und seine respiratorische Variation, bei speziellen Lungen- und Brustwanderkrankungen und Pleuraergüssen, ist noch begrenzt. In vielfaltigen Studien konnte aber aufgezeigt werden, dass selbst unter diesen Bedingungen der Ösophagusdruck ein akzeptabler, zielführender und geeigneter Surrogatparameter zum Pleuraldruck (63) darstellt. Für das zwischenzeitlich am häufigsten eingesetzte Peso-Messverfahren wird ein luft- oder gas-gefüllter Ösophagusballon, quasi eine modifizierte Magensonde verwendet, der über einem langen dünnen Katheter mit einem Druckaufnehmer verbunden ist. Ösophaguskatheter können an moderne Intensivventilatoren (20) mit entsprechenden Anschlüssen (23, 24), an spezielle Monitore (51) zur Ösophagusdruckmessung oder an die invasive RR-Messung von Intensivüberwachungsmonitoren angeschlossen werden.

Um eine zuverlässige Peso-Messung zu erhalten, muss sich der Ösophagusballon an einer geeigneten Position befinden und mit einem ausreichenden Luftvolumen befüllt sein. Bei Unterfüllung des Ballons wird der Peso nicht korrekt übertragen. Allerdings kann ein überfüllter Ballon auch zur Überschätzung des Drucks fuhren. Welches Luftvolumen für die Füllung "optimal" ist, hangt von Design, Abmessungen, Geometrie und Material des Ösophagusballons ab, die sich wiederum auf dessen mechanische Eigenschaften auswirken. Die sechs am weitesten verbreiteten handelsüblichen Ösophaguskatheter sind in vitro beim externen Drücken von 0 bis 30 cm H2O getestet worden. Dabei zeigte sich, dass alle untersuchten Katheter den Umgebungsdruck korrekt maßen, aber erhebliche Unterschiede im optimalen Füllvolumen zwischen den Kathetern bestanden.
Außerdem war das für brauchbare Messungen erforderliche Mindestvolumen grösser als zuvor empfohlen und hing überdies vom Umgebungsdruck ab. Auch eine weitere Studie zeigte, dass das Füllvolumen für die verschiedenen untersuchten Katheter unterschiedlich war, und dass bei hohen Drücken ein größeres Füllvolumen erforderlich war. In der klinischen Praxis lässt sich das optimale Füllvolumen beispielsweise einfach bestimmen, indem der Ballon nach und nach, im für den jeweiligen Katheter vorgesehenen Bereich, befüllt wird und so das geringste Volumen bestimmt wird, bei dem die gröbste Pulsdruckvariation ("Swing") des Peso in einem Atemzug (von inspiratorisch zu exspiratorisch) auftritt. Erfindungsgemäße Peso- Überwachungsmonitore und Intensivventilatoren verfügen über spezielle Algorithmen zur optimalen Füllung des Katheterballons.

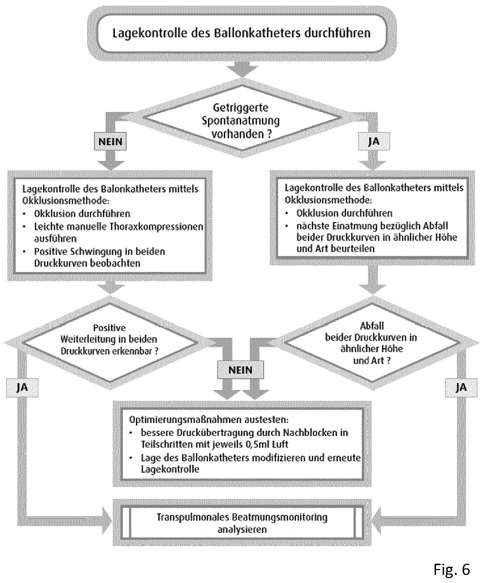

Fig. 6 zeigt die Lagekontrolle des Peso-Katheters. Nach dem Entleeren wird der Ösophagusballon mit einem Dreiwegehahn gesichert und entlang des Führungsdrahtes ein geeignetes Gleitmittel appliziert.
Vergleichbar einer Magensonde wird der Peso-Katheter vorsichtig bis zu einer Tiefe von etwa 55 cm bis in den Magen vorgeschoben und dann mit dem vom Hersteller empfohlenen Mindestvolumen befüllt. Die intra-gastrische Katheterposition wird durch eine leichte externe manuelle gastrische Kompression bestätigt, die einen positiven Druckausschlag hervorruft. Anschließend wird der Katheter nach und nach in den Ösophagus zurückgezogen.
Eine Positionierung im Ösophagus ist erkennbar am Auftreten kardialer Artefakte in der Druckmessung und durch den Wechsel von intra-abdominalen zu intra-thorakalen Druckverlaufsmustern. Durch die Platzierung des Ösophagusballons innerhalb der unteren zwei Drittel des intra-thorakalen Ösophagus sollen sich mögliche Druckartefakte aufgrund der inhomogenen Kompression des Ösophagus durch externe Strukturen vermeiden lassen. Wenn spontane inspiratorische Bemühungen vorhanden sind, besteht der herkömmliche Test zur Validierung der Peso-Messung darin, die gleichzeitigen negativen Ausschläge des Atemwegs- und Ösophagusdrucks während eines endexspiratorischen Okklusionsmanövers zu vergleichen (sogenannter Baydur-Test). Während der okkludierten Inspiration sollten die Druckänderungen im Atemweg (ΔPaw) und im Ösophagus (ΔPeso) fast identisch sein, weil sich das Lungenvolumen nicht ändert und damit auch keine Änderung im PL auftritt. Die Peso-Messung gilt als verlässlich, wenn das Verhältnis ΔPeso/ΔPaw zwischen 0,8 und 1,2 liegt. Andernfalls muss der Katheter neu positioniert bzw. das Ballonvolumen erneut überprüft werden.

Bei mandatorisch beatmeten Patienten ist eine externe manuelle Thoraxkompression während einer exspiratorischen Pause (Hold-Manöver) anzuwenden, während der, die gleichzeitigen positiven Ausschlage des Atemwegs- und des Ösophagusdrucks zu vergleichen sind (Überdruck- Okklusionstest). Bei gleicher Positionierung des Peso-Katheters liefern der Baydur-Test und der Überdruck-Okklusionstest ähnliche Druckänderungen in den Atemwegen und im Ösophagus. Der absolute Wert kann erheblich hoher sein, wenn der Ösophagusballon im unteren Drittel des Ösophagus anstatt im mittleren Drittel positioniert wird, weil hier der überlagerte Druck durch Herz und Lunge hoher ist. Außerdem kann der Druck, der von der Ösophaguswand als Reaktion auf die Ballonbefüllung erzeugt wird, den absoluten Wert des Peso über den des Pleuraldrucks erhöhen. Ein erfindungsgemäßes Kalibrierverfahren zur Beseitigung dieses Artefakts ist, dass die Verwendung des absoluten Werts des Ösophagusdruckes verbessert ist, insbesondere, wenn Ballons mit großem Volumen eingesetzt werden.

### Optimierung der Beatmung von Patienten

Der Nutzen der Ösophagusmessung als Basis der Beatmungstherapie beim ARDS-Patienten zeigt sich exemplarisch in einer Ösophagusdruckgesteuerten Beatmung.
Die PEEP-Werte werden mit dem Ziel eingestellt, einen transpulmonalen Druck (64) zwischen 0 und 10 cmH2O in der endexspiratorischen Phase gemäß einer gleitenden Skala basierend auf dem Verhältnis PaO2/FIO2 zu erreichen. Das Tidalvolumen wird beschränkt, um den transpulmonalen Druck in der endinspiratorischen Phase unter 25 cmH2O zu halten. Somit kann die PEEP-Optimierung auf Basis des Peso-Monitorings eingesetzt werden, um das Auftreten eines Atelekttraumas zu minimieren, die Oxygenierung zu optimieren und die Compliance des respiratorischen Systems zu verbessern.
Der endexspiratorische transpulmonale Druck (64, TPP exsp) (= Alveolardruck (65) - Pleuraldruck (63)) kann dann durch die Titration des angewandten PEEP angepasst werden, da der Atemwegsdruck mit dem angewandten PEEP in Zusammenhang steht. Die Titration des angewandten PEEP auf einen endexspiratorischen transpulmonalen Druck zwischen 0 und 10 mbar, aber zumindest im positiven Bereich, kann den zyklischen Alveolarkollaps verringern. Der endinspiratorische transpulmonale Druck TPP insp (Plateaudruck-Pleuraldruck) kann die alveoläre Überdehnung reduzieren und damit die Optimierung des eingestellten Tidalvolumens bzw. des inspiratorischen Drucks unterstützen. Als Zielbereich gilt, je nach klinischem Befund, ein Bereich von bis 20 mbar.

**Tab. 2: PEEP-Optimierung und Vermeidung der Überdehnung von Lungenarealen**

| **Abkürzung** | **Definition** | **Klinische Bedeutung** | **Klinische Empfehlung** |
|---|---|---|---|
| (64) TPP exp. | Endexspiratorischer transpulmonaler Druck (PEEP - Peso) | Atemzyklischer Alveolarkollaps | im positiven Bereich |
| (64) TPP insp. | Endinspiratorischer transpulmonaler Druck (Plateaudruck - Peso) | alveoläre Überdehnung | < 25 mbar |
| Δ PESO ΔTPP | Driving Pressure Ösophagus (ΔPeso = | • Unabhängiger Mortalitätsfaktor | < 12 mbar |
| | VT / Compliance) | • Maß für den tidalen Stress des Lungenparenchyms | < 12 mbar |
| | Transpulmonary Driving Pressure (TPP exp - TPP insp) | • Verhältnis von Tidalvolumen zu (statischer) respiratorischer Compliance | |

### Der Weaningprozesses unter Peso-Monitoring

Die Messung der Atemarbeit zur Quantifizierung der Atemanstrengung ermöglichen es Peso-Messungen, den Grad der Muskelentlastung unter Beatmung individuell an den Patienten anzupassen. Hierdurch kann eine erhöhte Atemmuskelanstrengung mit der Gefahr der respiratorischen Erschöpfung erkannt und in der Folge die Gefahr eines Weaningversagens minimiert werden. Dabei kann die inspiratorische Atemarbeit sowohl als Flächenintegral über die Zeit (PTP = Druck-Zeit-Produkt) als auch über das Volumen (WOB = Work of Breathing) ermittelt werden. Beide Messverfahren sind wirkungsvolle Methoden zur Einschätzung der Verlustleistung oder des Energieverbrauchs der Atemmuskulatur. Die Arbeit wird als Kraft x Verdrängung ausgedrückt. Bei einem spontan atmenden Patienten ist die von den Atemmuskeln erbrachte Arbeit gleich dem Integral des Produkts aus Pmus und Volumenänderung. Wenn Volumen erzeugt wird, besteht normalerweise eine enge Korrelation zwischen WOB und PTP.
Die WOB pro Atemzyklus wird normalerweise in Joule ausgedrückt. Arbeit pro Minute wird durch Multiplizieren von WOB pro Zyklus mit der entsprechenden Atemfrequenz berechnet. Arbeit pro Liter wird durch Dividieren von Arbeit pro Minute durch Minutenvolumen berechnet. Ein Joule ist die Arbeit, die erforderlich ist, um 1000ml Tidalvolumen über eine Druckdifferenz von 10 cm H2O zu bewegen. Durch diese Festlegungen kann ein orientierender Normwertbereich festgelegt werden.
Mehrere Studien konnten zeigen, dass sich die Atemanstrengung erhöht, wenn Patienten bei einem Entwöhnungsversuch versagen. Im Verlauf einer Studie zur Spontanatmung blieb das PTP bei der erfolgreichen Entwöhnung von Patienten unverändert. Dagegen entwickelten Patienten mit fehlgeschlagener Entwöhnung als Folge einer Erhöhung der mechanischen Belastung der Atemmuskulatur eine deutliche und progressive Erhöhung von PTP. Am Ende der Studie erhöhte sich der PTP der nicht entwöhnten Patienten um mehr als das Vierfache des normalen Werts. Im Verlauf eines fehlgeschlagenen Entwöhnungsversuchs wiesen Peso-Schwankungen größere Veränderungen auf als der Rapid Shallow Breathing Index (RSBI).
Mit der Unterscheidung von resistiver und elastischer Atemarbeit ergeben sich neue Ansätze die Art der unterstützenden Beatmung selektiv auszurichten, dass die Atemarbeit reduziert wird.

**Tab. 3: Weaning**

| **Abkürzung** | **Definition** | **Klinische Bedeutung** | **Klinische Empfehlung** |
|---|---|---|---|
| WOB | Work of Breathing: Maß für die inspiratorische Atemarbeit pro Atemzug Flächenintegral über das Volumen | Gefahr der respiratorischen Erschöpfung | 0,3-0,6 Joule/Liter |
| WOB/min. | Maß für die inspiratorische Atemarbeit pro Minute (Integral über das Volumen) übertragen auf das Atemminutenvolumen | Gefahr der respiratorischen Erschöpfung | 2,4 Joule pro Minute |
| PTPes | Pressure Time Product: Maß für die Atemanstrengung der Atemmuskulatur Flächenintegral über die Zeit (=Druck-Zeit-Produkt) | Gefahr der respiratorischen Erschöpfung | > 150 cmH2O*s |

### Messung des funktionellen oder intrinsischen PEEP

Bei chronisch obstruktiven Lungenerkrankungen kann es zu Obstruktionen in den kleinen Atemwegen kommen. Die damit einhergehende inkomplette Ausatmung des eingeatmeten Atemzugvolumens führt zu einer konsekutiver Überblähung der Lungen ("intrinsischer PEEP"). In ähnlicher Wiese kann ein vergleichbares Problem auch durch eine inadäquate Einstellung der Ausatemzeit verursacht werden. Das nicht abgeatmete Lungenvolumen erzeugt einen funktionellen positiven endexspiratorischen Druck, welcher als intrinsische PEEP (PEEPi) bezeichnet wird. Die Höhe des PEEPi ist ein valider Indikator für die dynamische Überblähung der COPD-Lunge.
Der jeweilige intrinsische PEEP muss bei jeder Einatmung von der Inspirationsmuskulatur durch entsprechende Pleuradrucknegativierung erst überwunden werden, ehe ein inspiratorischer Atemgasfluss zustande kommt. Die hierfür notwendige Atemarbeit wird als "isometrische Atemarbeit" bezeichnet und kann in Abhängigkeit des Schwere-grades der COPD mehr als die Hälfte der gesamten Atemarbeit ausmachen.

**Tab.4: Spontanatmung**

| **Abkürzung** | **Definition** | **Klinische Bedeutung** | **Klinische Empfehlung** |
|---|---|---|---|
| Peso min. | Geringster Druck innerhalb eines Atemzyklus | Maß für inadäquate Spontanatmung (z.B. beim ARDS) | relativ <10 mbar |
| Δ PEEP / Peso min. | Druckdelta zwischen PEEP und Peso min in der Inspiration | Maß für inädaquate Spontanatmung (z.B. beim ARDS) | < 10 mbar |

Im Gegensatz zur PEEPi-Messung mittels eines speziellen Manöver des Beatmungsgerätes kann unter Spontanatmung der intrinsische PEEP bei korrekt liegendem Peso-Katheter fortlaufend gemessen werden. Dabei wird der flussunwirksame Abfall des Ösophagusdrucks während der Inspiration gemessen, welcher ein Surrogatparameter für den PEEPi ist. Darauf aufbauend kann das Trappingvolumen kalkuliert werden.
Erfindungsgemäß kann der optimale PEEP auch durch Ermittlung des transpulmonalen Druckes gefunden werden. Der transpulmonale Druck wird als Differenz zwischen Alveolardruck und Pleuradruck bestimmt, wobei der Pleuradruck durch Messung des Peso-Drucks (60) abgeschätzt wird. Erfindungsgemäß wird nur bei positivem endexspiratorischem transpulmonalen Druck ein Alveolarkollaps in der Exspiration verhindert. Entsprechend erfolgt die Drucksteuerung derart, dass end-exspiratorisch ein positiver transpulmonaler Druck vorherrscht.

**Tab. 5: Inadäquate Exspiration**

| **Abkürzu ng** | **Definition** | **Klinische Bedeutung** | **Klinische Empfehlung** |
|---|---|---|---|
| PEEPi. | Intrinsisc her PEEP | Maß für den dynamischen PEEP (Auto-PEEP) | Kontrolle & ggfs. Adaption: |
| | | | • Inspirationszeit |
| | | | • Exspirationszeit |
| | | | • Frequenz |
| | | | • Druckanstiegsgeschwindigkeit ("Rampe") |
| | | | • Inspiratorische Terminierung ("Flowabschaltkriterium" z.B. "PSV-Endflow") |
| | | | • PEEP |
| | | | • Höhe der Druckunterstützung |
| Vtrap. | Trappingvo lumen | Indikator für alveoläre Überdehnung ("Überblähung") | Kontrolle & ggfs. Adaption: |
| | | | • Inspirationszeit |
| | | | • Exspirationszeit |
| | | | • Frequenz |
| | | | • Druckanstiegsgeschwindigkeit ("Rampe") |
| | | | • Inspiratorische Terminierung ("Flowabschaltkriterium" z.B. "PSV-Endflow") |
| | | | • PEEP |
| | | | • Höhe der Druckunterstützung |

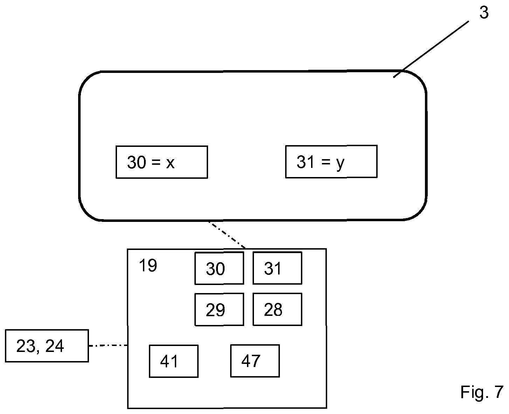

Figur 7 veranschaulicht die Ermittlung einer Fehltriggerrate. Das System weist zudem eine Einrichtung zur Erfassung der Synchronität (28) eines durch das Beatmungsgerät (20) zeitlich vorgegebenen Atemgashubs (47) (Druck oder Volumen oder Fluss) mit der Atemanstrengung (41) des Patienten (40) auf. Die Einrichtung zur Erfassung der Synchronität (28) kann Teil der Steuereinheit (19) sein.
Aus der Ösophagusdruckkurve (60) wird die Anzahl der Inspirationsbemühungen (41) des Patienten (pro Zeiteinheit) erkannt und aufgezeichnet.
Aus der Beatmungsdruckkurve (27) oder entsprechend der Vorgabewerte der Steuereinheit (bei kontrollierter Beatmung) wird die Anzahl der vorgegebenen Inspirationen (47) (pro Zeiteinheit) erkannt oder entnommen und aufgezeichnet.

Die Inspirationsbemühungen des Patienten (41) (pro Zeiteinheit) werden mit den vorgegebenen Inspirationen (47) (pro Zeiteinheit) verglichen und so der Grad der Synchronisierung (30) zwischen den Inspirationsbemühungen (41) des Patienten und der Inspiratorischen Vorgabe (47) des Beatmungsgeräts ermittelt.
Diesem Vergleich kann ein Index (31) der Fehltriggerungen entnommen werden, der den Grad der Synchronisierung (30) zwischen den Inspirationsbemühungen des Patienten und der Inspiratorischen Vorgabe des Beatmungsgeräts repräsentiert. Der Index (31) und/oder der Grad der Synchronisierung (3) können beispielsweise auf der Anzeigeeinheit (3) des Beatmungsgerätes oder auf einem Monitor ausgegeben werden. Der Index (31) und/oder der Grad der Synchronisierung (30) können alternativ oder ergänzend auch für die Steuerung der Beatmung von der Steuereinheit (19) zumindest teilweise berücksichtigt werden. Beispielsweise indem bei der kontrollierten Beatmung die Frequenz der Atemhübe (47) unter Berücksichtigung von Index (31) und/oder Grad der Synchronisierung (30) automatisch angepasst werden.
Das Beatmungsgerät oder ein Monitor weisen eine Einrichtung zur Erfassung der Synchronität (28) auf, die als Teil der Steuereinheit (19) ausgeführt sein kann, die Fehltrigger (31), aus dem zeitlichen Abstand (29) zwischen dem vorgegebenen Atemgashub (47) und der Atemanstrengung (41) des Patienten (40) erkennt und eine Fehltriggerrate oder ein Index (31) ermittelt und speichert oder darstellt.
Die Einrichtung zur Erfassung der Synchronität (28) ermittelt Fehltrigger (31), aus dem zeitlichen Abstand zwischen dem vorgegebenen Atemgashub (47) und der Atemanstrengung (41) des Patienten dann, wenn der zeitliche Abstand (29) größer ist als 1/100 Sekunde, bevorzugt größer ist 1/10 Sekunde, besonders bevorzugt größer ist als eine Sekunde.

## Patentansprüche

1. System zur Erfassung von Atemanstrengungen (41) eines Patienten (40), umfassend eine Druckermittlungsvorrichtung (61, 62) zum Ermitteln eines Druckes (60, 64) zu dem Zeitpunkt (50) der Atemanstrengung (41) des Patienten (40), wobei die Druckermittlungsvorrichtung als Ösophagus-Katheter (61) ausgebildet ist und einen gasgefüllten Ballon (62) aufweist und umfassend ein Beatmungsgerät (20) zur unterstützenden Beatmung eines Patienten mit einem Druckeingangsstutzen (23) für den Ösophagus-Katheter (61) und einen Drucksensor (24), wobei eine Atemanstrengung (41) des Patienten (40) über den gasgefüllten Ballon (62) des Ösophagus-Katheters (61) erfasst wird wobei der Ösophagus-Katheter (61) den funktionellen positiven endexspiratorischen Druck, welcher als intrinsischen PEEP (PEEPi) bezeichnet wird, unter Spontanatmung ermittelt, wobei das System zudem eine Einrichtung, beispielsweise eine Steuereinheit, umfasst, die derart konfiguriert ist, dass sie, zur Erfassung der Synchronität (28) einen, durch das Beatmungsgerät (20), zeitlich vorgegebenen Atemgashub (47), wie Druck oder Volumen oder Fluss, mit der Atemanstrengung (41) des Patienten (40) vergleicht,
**dadurch gekennzeichnet, dass** mit diesem Vergleich ein Index (31) der Fehltriggerungen ermittelt und dargestellt wird, der den Grad der Synchronisierung (30) zwischen den Inspirationsbemühungen des Patienten und der inspiratorischen Vorgabe des Beatmungsgeräts repräsentiert.

2. System nach Anspruch 1, **dadurch gekennzeichnet, dass** der Sensor (24) des Beatmungsgerätes (20) den Ösophagusdruck (60) bestimmt, der über den gasgefüllten Ballon (62) des Ösophagus-Katheters (61) erfasst wird.

3. System nach zumindest einem der vorhergehenden Ansprüche **dadurch gekennzeichnet, dass** der transpulmonale Druck (64) von dem Beatmungsgerät bestimmt wird unter Berücksichtigung des, vom Beatmungsgerät vorgegebenen, Beatmungsdrucks (27) und dem sensorisch ermittelten Ösophagusdruck (60).

4. System nach zumindest einem der vorhergehenden Ansprüche **dadurch gekennzeichnet, dass** der gasgefüllte Ballon (62) einen Sensor aufweist, der eine Atemanstrengung (41) registriert und an ein Beatmungsgerät übermittelt.

5. System nach zumindest einem der vorhergehenden Ansprüche **dadurch gekennzeichnet, dass** die Bestimmung des transpulmonalen Drucks (64) am Ende der Ausatmung (64, TPP ex) und/oder am Ende der Einatmung (64, TPP in) durch einen bestimmten Messvorgang erfolgt, bei dem das Beatmungsgerät einen Atemgastransport zu oder von dem Patienten verhindert.

6. System nach zumindest einem der vorhergehenden Ansprüche **dadurch gekennzeichnet, dass** eine Atemanstrengung (41) des Patienten (40) mit zumindest einem hinterlegten Schwellwert verglichen wird und bei Überschreitung des Schwellwertes einem Trigger (42) entspricht.

7. System nach zumindest einem der vorhergehenden Ansprüche **dadurch gekennzeichnet, dass** der Zeitpunkt (50) der Beginn der Exspiration oder der Beginn der Inspiration (41) des Patienten ist.

8. System nach zumindest einem der vorhergehenden Ansprüche **dadurch gekennzeichnet, dass** die Einrichtung (19) den, von dem Beatmungsgerät (20) bereitgestellten, Atemgasdruck unter Berücksichtigung des ermittelten Ösophagusdrucks (60) oder transpulmonalen Drucks (64) vorgibt.

9. System nach zumindest einem der vorhergehenden Ansprüche **dadurch gekennzeichnet, dass** die Einrichtung (19) bei Überschreiten oder Unterschreiten eines Schwellwertes für den Ösophagusdruck (60) ein Steuersignal für das Beatmungsgerät (20) erzeugt, zur Vorgabe eines inspiratorischen oder exspiratorischen Atemgasdrucks (47).

10. System nach zumindest einem der vorhergehenden Ansprüche **dadurch gekennzeichnet, dass** aus der Ösophagusdruckkurve (60) die Anzahl der Inspirationsbemühungen (41) des Patienten pro Zeiteinheit erkannt und aufgezeichnet wird und/oder, dass aus der Beatmungsdruckkurve (27) oder der Steuereinheit die Anzahl der vorgegebenen Inspirationen (47) pro Zeiteinheit erkannt und aufgezeichnet wird.

11. System nach zumindest einem der vorhergehenden Ansprüche **dadurch gekennzeichnet, dass** die Inspirationsbemühungen des Patienten (41) pro Zeiteinheit mit den vorgegebenen Inspirationen (47) pro Zeiteinheit verglichen und so der Grad der Synchronisierung (30) zwischen den Inspirationsbemühungen (41) des Patienten und der Inspiratorischen Vorgabe des Beatmungsgeräts ermittelt wird.

12. System nach zumindest einem der vorhergehenden Ansprüche **dadurch gekennzeichnet, dass** die Einrichtung (19) bei erkannten Fehltriggern (31) ein Steuersignal für das Beatmungsgerät (20) erzeugt, zur Vorgabe einer veränderten Einatemzeit und/oder eines veränderten PEEP und/oder einer veränderten Triggerempfindlichkeit.

13. System nach zumindest einem der vorhergehenden Ansprüche **dadurch gekennzeichnet, dass** die Einrichtung (19) bei Überschreiten oder Unterschreiten eines Schwellwertes für den PEEPi ein Steuersignal für das Beatmungsgerät (20) erzeugt, zur Vorgabe eines veränderten Atemgasvolumens oder einer veränderten Ausatemzeit.

14. System nach zumindest einem der vorhergehenden Ansprüche **dadurch gekennzeichnet, dass** eine Einrichtung zur Erfassung des optimalen (70) Füllvolumens des Ballons (62) umfasst ist, wobei die Einrichtung das geringste Füllvolumen bestimmt, welches die größte Pulsdruckvariation , wie ("Swing" , des Ballons (62) in einem Atemzug von inspiratorisch zu exspiratorisch ausmacht .

## Claims

1. A system for detecting the respiratory effort (41) of a patient (40), comprising a pressure determination device (61, 62) for determining a pressure (60, 64) at the point in time (50) of the respiratory effort (41) of the patient (40), wherein the pressure determination device is designed as an esophageal catheter (61) and has a gas-filled balloon (62), and comprising a respirator (20) for assisted respiration of a patient with a pressure inlet nozzle (23) for the esophageal catheter (61) and a pressure sensor (24), wherein a respiratory effort (41) of the patient (40) is detected via the gas-filled balloon (62) of the esophageal catheter (61), wherein the esophageal catheter (61) determines the functional positive end-expiratory pressure, which is referred to as intrinsic PEEP (PEEPi), during spontaneous. respiration, wherein the system additionally comprises an apparatus, for example a control unit, which is configured in such a way that, in order to detect synchronicity (28), it compares a respiratory gas stroke (47) chronologically specified by the respirator (20), such as pressure or volume or flow, with the respiratory effort (41) of the patient (40), **characterized in that** an index (31) of the failed triggers is determined and constituted with the comparison, which index represents the degree of the synchronization (30) between the inspiration efforts of the patient and the inspiration specification of the respirator.

2. The system according to Claim 1, **characterized in that** the sensor (24) of the respirator (20) establishes the esophageal pressure (60) which is detected via the gas-filled balloon (62) of the esophageal catheter (61).

3. The system according to at least one of the preceding claims, **characterized in that** the transpulmonary pressure (64) is established by the respirator in consideration of the respiration pressure (27) specified by the respirator and the esophageal pressure (60) determined by the sensor.

4. The system according to at least one of the preceding claims, **characterized in that** the gas-filled balloon (62) has a sensor which registers a respiratory effort (41) and transmits it to a respirator.

5. The system according to at least one of the preceding claims, **characterized in that** the establishment of the transpulmonary pressure (64) is performed at the end of the exhalation (64, TPP ex) and/or at the end of the inhalation (64, TPP in) by a specific measuring procedure, in which the respirator prevents a respiratory gas transportation to or from the patient.

6. The system according to at least one of the preceding claims, **characterized in that** a respiratory effort (41) of the patient (40) is compared to at least one stored threshold value and corresponds to a trigger (42) if the respiratory effort exceeds the threshold value.

7. The system according to at least one of the preceding claims, **characterized in that** the point in time (50) is the beginning of the expiration or the beginning of the inspiration (41) of the patient.

8. The system according to at least one of the preceding claims, **characterized in that** the apparatus (19) specifies the respiratory gas pressure provided by the respirator (20) in consideration of the determined esophageal pressure (60) or transpulmonary pressure (64).

9. The system according to at least one of the preceding claims, **characterized in that**, if the esophageal pressure (60) exceeds or falls below a threshold value, the apparatus (19) generates a control signal for the respirator (20) to specify an inspiratory or expiratory respiratory gas pressure (47).

10. The system according to at least one of the preceding claims, **characterized in that** the number of inspiration efforts (41) of the patient per unit of time is recognized and recorded from the esophageal pressure curve (60) and/or the number of specified inspirations (47) per unit of time is recognized and recorded from the respiration pressure curve (27) or the control unit.

11. The system according to at least one of the preceding claims, **characterized in that** the inspiration efforts of the patient (41) per unit of time are compared to the specified inspirations (47) per unit of time and the degree of synchronization (30) between the inspiration efforts (41) of the patient and the inspiration specification of the respirator is thus determined.

12. The system according to at least one of the preceding claims, **characterized in that** the apparatus (19) generates a control signal for the respirator (20) to specify a changed inhalation time and/or a changed PEEP and/or a changed trigger sensitivity if failed triggers (31) are recognized.

13. The system according to at least one of the preceding claims, **characterized in that**, if the PEEPi exceeds or falls below a threshold value, the apparatus (19) generates a control signal for the respirator (20) to specify a changed respiratory gas volume or a changed exhalation time.

14. The system according to at least one of the preceding claims, **characterized in that** the system comprises an apparatus for detecting the optimum (70) filling volume of the balloon (62), wherein the apparatus establishes the lowest filling volume which makes up the greatest pulse.pressure variation ("swing") of the balloon (62) in one breath from inspiratory to expiratory.

## Revendications

1. Système de détection des efforts respiratoires (41) d'un patient (40), comprenant un dispositif de mesure de pression (61, 62) servant à déterminer une pression (60, 64) à l'instant (50) de l'effort respiratoire (41) du patient (40), dans lequel le dispositif de mesure de pression est conçu sous la forme d'un cathéter œsophagien (61) et présente un ballonnet rempli de gaz (62) et comprenant un respirateur (20) destiné à la ventilation assistée d'un patient avec une tubulure d'entrée de pression (23) du cathéter œsophagien (61) et un capteur de pression (24), dans lequel un effort respiratoire (41) du patient (40) est détecté par le biais du ballonnet rempli de gaz (62) du cathéter œsophagien (61), dans lequel le cathéter œsophagien (61) détermine la pression positive fonctionnelle en fin d'expiration, laquelle est désignée par l'abréviation PEEP intrinsèque (PEEPi), lors d'une respiration spontanée, dans lequel le système comprend en outre un appareil, par exemple une unité de commande, qui est configuré de telle manière qu'il compare une élévation du gaz respiratoire (47) préétablie dans le temps par le respirateur (20), telle que la pression ou le volume ou le débit, avec l'effort respiratoire (41) du patient (40) pour détecter le synchronicité (28), **caractérisé en ce que** la comparaison permet de déterminer et de représenter un indice (31) des déclenchements erronés, qui met en évidence le degré de synchronisation (30) entre les efforts inspiratoires du patient et la consigne inspiratoire du respirateur.

2. Système selon la revendication 1, **caractérisé en ce que** le capteur (24) du respirateur (20) définit la pression œsophagienne (60), qui est détectée par le biais du ballonnet rempli de gaz (62) du cathéter œsophagien (61).

3. Système selon l'une au moins des revendications précédentes, **caractérisé en ce que** . la pression transpulmonaire (64) du respirateur est définie compte tenu de la pression ventilatoire (27) préétablie par le respirateur et de la pression œsophagienne (60) déterminée par le capteur.

4. Système selon l'une au moins des revendications précédentes, **caractérisé en ce que** le ballonnet rempli de gaz (62) présente un capteur, qui enregistre un effort respiratoire (41) et le transmet à un respirateur.

5. Système selon l'une au moins des revendications précédentes, **caractérisé en ce que** l'évaluation de la pression transpulmonaire (64) à la fin de l'expiration (64, TPP ex) et / ou à la fin de l'inspiration (64, TPP in) est réalisée moyennant un processus de mesure bien défini, au cours duquel le respirateur empêche un transport de gaz respiratoire à destination ou en provenance du patient.

6. Système selon l'une au moins des revendications précédentes, **caractérisé en ce qu'**un effort respiratoire (41) du patient (40) est comparé avec au moins une valeur de seuil mémorisée et équivaut à un déclenchement (42) en cas de dépassement de la valeur de seuil.

7. Système selon l'une au moins des revendications précédentes, **caractérisé en ce que** l'instant (50) est le début de l'expiration ou le début de l'inspiration (41) du patient.

8. Système selon l'une au moins des revendications précédentes, **caractérisé en ce que** l'appareil (19) préétablit la pression de gaz respiratoire fournie par le respirateur (20) compte tenu de la pression œsophagienne (60) ou de la pression transpulmonaire (64) déterminées.

9. Système selon l'une au moins des revendications précédentes, **caractérisé en ce que** l'appareil (19) génère un signal de commande destiné au respirateur (20) si la pression œsophagienne (60) est supérieure ou inférieure à une valeur de seuil, afin de préétablir une pression de gaz respiratoire d'inspiration ou d'expiration (47).

10. Système selon l'une au moins des revendications précédentes, **caractérisé en ce que** le nombre des efforts inspiratoires (41) du patient par unité de temps est décelé et enregistré à partir de la courbe de la pression œsophagienne (60) et / ou que le nombre des inspirations préétablies (47) par unité de temps est décelé et enregistré à partir de la courbe de la pression ventilatoire (27) ou de l'unité de commande.

11. Système selon l'une au moins des revendications précédentes, **caractérisé en ce que** les efforts inspiratoires du patient (41) par unité de temps sont comparés avec les inspirations préétablies (47) par unité de temps et le degré de synchronisation (30) entre les efforts inspiratoires (41) du patient et la consigne inspiratoire du respirateur est ainsi déterminé.

12. Système selon l'une au moins des revendications précédentes, **caractérisé en ce que** l'appareil (19) génère un signal de commande destiné au respirateur (20) si des déclenchements erronés (31) sont décelés, afin de préétablir un temps d'inspiration modifié et / ou une PEEP modifiée et / ou une sensibilité de déclenchement modifiée.

13. Système selon l'une au moins des revendications précédentes, **caractérisé en ce que** l'appareil (19) génère un signal de commande destiné au respirateur (20) si la PEEPi est supérieure ou inférieure à une valeur de seuil, afin de préétablir un volume de gaz respiratoire modifié ou un temps d'expiration modifié.

14. Système selon l'une au moins des revendications précédentes, **caractérisé en ce qu'**un appareil de détection du volume de remplissage optimal (70) du ballonnet (62) est inclus, dans lequel l'appareil définit le volume de remplissage le plus faible, lequel constitue la plus grande variation de pression différentielle telle que la modulation « swing » du ballonnet (62) au cours d'un cycle respiratoire de l'inspiration jusqu'à l'expiration.
